# EUROPEAN PATENT APPLICATION

(11) **EP 3 842 109 A1**
(43) Date of publication of application: **30.06.2021**
(21) Application number: 19306756.8
(22) Date of filing: 23.12.2019
(51) Int. Cl.: B01D 19/04, C07C 29/34, C07C 31/125

(54) **ANTIFOAM AND DEFOAMER PRODUCT**

(71) Applicant: Oleon N.V., 9940 Ertvelde (BE)
(72) Inventor: KERBRAT, Marion, 60280 MARGNY-LES-COMPIEGNE (FR); ZITOUNI, Karima, 91420 MORANGIS (FR); HUART, Cyrielle, 60750 Choisy au Bac (FR)
(74) Representative: Santarelli

(57) **Abstract**

The present invention relates to a combination of specific alcohol and ester and their use as antifoam and/or defoamer. It also concerns compositions comprising it, and methods for reducing foam formation and for breaking a foam.

## Description

The present invention relates to a product and its use as antifoam and/or defoamer.

A foam is a dispersion of gas bubbles into a liquid.

Almost every industrial sectors are concerned by unwanted foam formation, such as food processing, paper industry, textile drying, paint manufacture, oil and gas industry and waste water treatment.

Unwanted foam can be formed during an industrial process, such as during the agitation of a composition comprising a compound capable of foaming the composition. This production of unwanted foam may lead to significant problems, such as reductions in product performance or productivity losses.

Thus, it is necessary to prevent or reduce the foaming or to break the foam.

Antifoams and defoamers are chemical compounds used for foam control. They both destabilize a foam.

An antifoam prevents or reduces the foam formation.

An defoamer breaks an existing foam.

Various antifoams and defoamers are currently available.

US 2014/0251605 A1 describes a method for defoaming a foam with a composition comprising at least an alcohol, linear or branched, represented by Figures I to IV. Based on those figures, the alcohol can potentially, comprise between 3 and 997 carbon atoms.

It has been surprisingly found that a combined preparation of specific branched alcohol and specific alkyl ester of branched alcohol presents a synergy in destabilization of foam, allowing reducing foam formation and/or breaking a foam faster.

The present invention therefore relates to a product comprising or consisting of:
- a beta-alkylated monohydric primary alcohol O1; and
- an alkyl ester of a beta-alkylated monohydric primary alcohol O2;
wherein each beta-alkylated monohydric primary alcohol O1 and O2, identical or different, comprises from 8 to 16 carbon atoms; and
wherein the alkyl of the alkyl ester of a beta-alkylated monohydric primary alcohol O2 comprises from 5 to 12 carbon atoms;
as a combined preparation for simultaneous or separate use.

The product according to the invention is a juxtaposition of separate but functionally interacting individual components, a specific beta-alkylated monohydric primary alcohol O1 and a specific alkyl ester of a beta-alkylated monohydric primary alcohol O2 ("kit-of-parts").

As illustrated in Example 2.2, the specific beta-alkylated monohydric primary alcohol O1 and the specific alkyl ester of a beta-alkylated monohydric primary alcohol O2 can be first combined together before their simultaneous introduction in a composition (Example 2.2.1) or introduced individually one by one in the composition (Example 2.2.2).

In the present application, unless otherwise indicated, all ranges of values used are to be understood as being inclusive limits.

Advantageously, the product of the invention is used to destabilize foam, in particular a foam in formation or already formed on an aqueous composition.

The beta-alkylated monohydric primary alcohol is a branched alcohol, with at least an alkyl group linked at the beta carbon or the 2^{nd} carbon of the monohydric primary alcohol, in compliance with UIPAC nomenclature.

The alkyl group consists of carbon and hydrogen atoms.

The alkyl group may be straight or branched, preferably saturated.

The alkyl ester of beta-alkylated monohydric primary alcohol comprises from 13 to 28 carbon atoms.

Advantageously, in the product of the invention, each beta-alkylated monohydric primary alcohols O1 and O2 are individually of Formula (I): 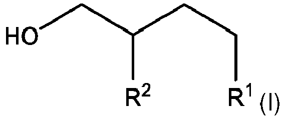 wherein R¹ and R², identical or different, are linear or branched alkyl group comprising from 2 to 6 carbon atoms.

The beta-alkylated monohydric primary alcohols O1 and O2 can be identical or different.

In a first embodiment, the beta-alkylated monohydric primary alcohols O1 and O2 are identical.

It means that for beta-alkylated monohydric primary alcohols O1 and O2 of Formula (I), alkyl groups R¹ and R² of O1 are identical to alkyl groups R¹ and R² of O2.

In a first particular embodiment of the first embodiment, R¹ and R² of O1 are identical to R¹ and R² of O2, and each R¹ is identical to R².

In a second particular embodiment of the first embodiment, R¹ and R² of O1 are identical to R¹ and R² of O2, even though each R¹ is different from R².

In a second embodiment, the beta-alkylated monohydric primary alcohols O1 and O2 are different.

It means that for beta-alkylated monohydric primary alcohols O1 and O2 of Formula (I), R¹ of O1 is different from R¹ of O2 and/or R² of O1 is different from R² of O2.

In a first particular embodiment of the second embodiment, R¹ and R² of O1, or R¹ and R² O2, are identical.

In a second particular embodiment of the second embodiment, all alky groups of O1 and O2 are different.

The beta-alkylated monohydric primary alcohols are preferably saturated.

The beta-alkylated monohydric primary alcohols are obtainable by a Guerbet reaction or by a process involving an hydroformylation reaction followed by an aldol condensation.

The Guerbet reaction is well known in the art. A Guerbet reaction comprises the condensation of two, identical or different, primary alcohols with elimination of water. It typically proceeds in the presence of a base such as NaOH or KOH and/or catalyst such as palladium, at a temperature of at least 120 °C.

In case of at least two different primary alcohols are used, then the Guerbet alcohol may be a mixture of Guerbet alcohols, having the same or different numbers of carbon atoms; i.e. a mixture of several beta-alkylated monohydric primary alcohols.

In particular, this corresponds to a mixture of beta-alkylated monohydric primary alcohols of Formula (I) with alkyls groups R¹ and R² different.

Preferably each beta-alkylated monohydric primary alcohol O1 and O2 is independently obtainable from primary alcohol monomer(s) chosen from the group consisting of butanol, 2-methylpropanol, pentanol, 3-methylbutanol, 2-methylbutanol, octanol and their mixtures.

Preferably, each beta-alkylated monohydric primary alcohol O1 and O2 are independently chosen from the group consisting of 2-ethylhexanol, 2-propylheptanol, 2-isopropyl-5-methyl-hexanol, 2-butyloctanol, 2-hexyloctanol and 2-hexyldecanol.

More preferably, the beta-alkylated monohydric primary alcohols O1 and O2 comprise, independently, from 8 to 14 carbon atoms.

More preferably, the beta-alkylated monohydric primary alcohols O1 and O2 comprise, independently, from 8 to 12, even more preferably from 8 to 10 carbon atoms.

In particular, each beta-alkylated monohydric primary alcohol O1 and O2 is independently chosen from the group consisting of 2-ethylhexanol, 2-propylheptanol and 2-isopropyl-5-methyl-hexanol.

An alkyl ester of beta-alkylated monohydric primary alcohol is obtainable by esterification reaction of a beta-alkylated monohydric primary alcohol with a carboxylic acid. The esterification reaction may be carried out according to any well-known conventional reaction conditions.

As an example, the esterification reaction may be carried out at a temperature comprised between 150 and 250 °C, preferably between 160 and 240°C.

An esterification catalyst may be used, such as methanesulfonic acid.

Advantageously, the water is removed as it forms from the reaction mixture.

Preferably, the esterification reaction is carried out until the acid value is less than 10, preferably less than 5 mg KOH/g, the acid value being measured according to standard AOCS Cd 3D-63.

Advantageously, in the product of the invention, R1 and R2 are identical in a beta-alkylated monohydric primary alcohol.

This is for example the case when the beta-alkylated monohydric primary alcohol of Formula (I) is obtained via a Guerbet reaction from identical primary alcohols.

Alternatively, the beta-alkylated monohydric primary alcohol of Formula (I) may be with R1 and R2 different.

This is the case when the beta-alkylated monohydric primary alcohol of Formula (I) is prepared from the condensation of two different primary alcohols via a Guerbet reaction.

Advantageously, in the product of the invention, the alkyl of the alkyl ester of beta-alkylated monohydric primary alcohol O2 comprises from 6 to 10 carbon atoms.

More preferably, the alkyl of the alkyl ester of beta-alkylated monohydric primary alcohol O2 comprises from 6 to 8, even more preferably 6 carbon atoms.

Preferably, the alkyl of the alkyl ester of beta-alkylated monohydric primary alcohol O2 is linear.

Advantageously, in the product of the invention, the total quantity of beta-alkylated monohydric primary alcohol(s) O1 is of at least 20% by weight, and the total quantity of alkyl ester(s) of beta-alkylated monohydric primary alcohol(s) O2 is of at least 20% by weight, weight percentages being based on the weight of the product.

By "total quantity of beta-alkylated monohydric primary alcohol(s) O1", it is intended the weight of all beta-alkylated monohydric primary alcohol O1 molecules comprising from 8 to 16 carbon atoms,

By "total quantity of alkyl ester(s) of beta-alkylated monohydric primary alcohol(s) O2", it is intended the weight of all alkyl ester(s) of beta-alkylated monohydric primary alcohol O2 molecules, wherein the beta-alkylated monohydric primary alcohol O2 comprises from 8 to 16 carbon atoms and the alkyl of the alkyl ester of beta-alkylated monohydric primary alcohol O2 comprises from 5 to 12 carbon atoms.

Advantageously, in the product of the invention, the ratio (total quantity of beta-alkylated monohydric primary alcohol(s) O1) / (total quantity of alkyl ester(s) of beta-alkylated monohydric primary alcohol(s) O2), is preferably comprised between 20/80 and 80/20.

Preferably, the ratio (total quantity of beta-alkylated monohydric primary alcohol(s) O1) / (total quantity of alkyl ester(s) of beta-alkylated monohydric primary alcohol(s) O2), is comprised between 25/75 and 75/25.

A particularly preferred product of the invention comprises or consists of:
- at least 20% by weight of beta-alkylated monohydric primary alcohol(s) O1; and
- at least 20% by weight of alkyl ester of beta-alkylated monohydric primary alcohol(s) O2;
wherein each beta-alkylated monohydric primary alcohol O1 and O2, identical or different, comprises from 8 to 10 carbon atoms;
wherein the alkyl the alkyl ester of beta-alkylated monohydric primary alcohol O2 comprises 6 carbon atoms; and
wherein the ratio (total quantity of beta-alkylated monohydric primary alcohol(s) O1 having from 8 to 10 carbon atoms) / (total quantity of alkyl ester(s) of beta-alkylated monohydric primary alcohol(s) O2 having from 8 to 10 carbon atoms and wherein the alkyl comprises 6 carbon atoms), is preferably comprised between 20/80 and 80/20.

In a preferred embodiment, components of the product of the invention are used simultaneously, for example in the form of an additive package.

The beta-alkylated monohydric primary alcohol O1 and the alkyl ester of beta-alkylated monohydric primary alcohol O2 components are first blended together to form an additive package.

Thus, the invention concerns an additive package comprising or consisting of:
- a beta-alkylated monohydric primary alcohol O1; and
- an alkyl ester of beta-alkylated monohydric primary alcohol O2;
wherein each beta-alkylated monohydric primary alcohol O1 and O2, identical or different, comprises from 8 to 16 carbon atoms, and
wherein the alkyl of the alkyl ester of beta-alkylated monohydric primary alcohol O2 comprises from 5 to 12 carbon atoms.

Alternatively, in another embodiment, components of the product of the invention are used separately. The beta-alkylated monohydric primary alcohol O1 and the alkyl ester of beta-alkylated monohydric primary alcohol O2 components are individually and/or successively introduced into a composition comprising a compound capable of foaming the composition.

The invention also relates to a process for preparing a product of the invention, by bringing together a beta-alkylated monohydric primary alcohol O1 and an alkyl ester of beta-alkylated monohydric primary alcohol O2, wherein each beta-alkylated monohydric primary alcohol O1 and O2, identical or different, comprises from 8 to 16 carbon atoms, and wherein the alkyl of the alkyl ester of beta-alkylated monohydric primary alcohol O2, comprises from 5 to 12 carbon atoms.

The beta-alkylated monohydric primary alcohol(s) O1 and the alkyl ester(s) of beta-alkylated monohydric primary alcohol(s) O2 are as described above, including preferential and advantageous features.

Preferably, the total quantity of beta-alkylated monohydric primary alcohol(s) O1 is of at least 20% by weight, and the total quantity of alkyl ester(s) of beta-alkylated monohydric primary alcohol(s) O2 is of at least 20% by weight, percentages by weight being based on the weight of the product.

Preferably, the ratio (total quantity of beta-alkylated monohydric primary alcohol(s) O1) / (total quantity of alkyl ester(s) of beta-alkylated monohydric primary alcohol(s) O2), is preferably comprised between 20/80 and 80/20, more preferably between 25/75 and 75/25.

In a first embodiment of the process of the invention, the components of the product are used simultaneously, in the form of an additive package, each component of the product are first brought together.

In a first particular embodiment of the first embodiment of the process of the invention, the bringing together is carried out by mixing of a beta-alkylated monohydric primary alcohol O1 and an alkyl ester of beta-alkylated monohydric primary alcohol O2, preferably at room temperature, in particular at 25°C +/-5°C.

In a second particular embodiment of the first embodiment of the process of the invention, the bringing together is carried out by partially esterifying the beta-alkylated monohydric primary alcohol O1 with a carboxylic acid.

In a second embodiment of the process of the invention, the components of the product are used separately, then one of them is first brought together with a third component.

In a particular embodiment of the second embodiment of the process of the invention, the beta-alkylated monohydric primary alcohol O1 or the alkyl ester of beta-alkylated monohydric primary alcohol O2, is first added to water, then the second component of the product of the invention is added.

The invention also relates to an use of the product of the invention, as an antifoam and/or a defoamer.

The beta-alkylated monohydric primary alcohol(s) O1 and the alkyl ester(s) of beta-alkylated monohydric primary alcohol(s) O2 are as described above, including preferential and advantageous features.

The combination of beta-alkylated monohydric primary alcohol O1 and alkyl ester of beta-alkylated monohydric primary alcohol O2, wherein each beta-alkylated monohydric primary alcohol O1 and O2, identical or different, comprises from 8 to 16 carbon atoms, and wherein the alkyl of the alkyl ester of beta-alkylated monohydric primary alcohol O2 comprises from 5 to 12 carbon atoms, allows to destabilize a foam.

More particularly, the combination reduces the foam formation, by at least 20% immediately at t=0 and by at least 80% in less than 10 min, as showed by different combinations tested in Example 2.

Moreover, this combination allows to defoam an existing foam. In particular, as it can be seen in Example 2.5, the combination break 50% of the volume of a foam in less than 8 min, preferably in less than 5 min, more preferably in less than 3 min.

Advantageously, in the use of the invention, the ratio (total quantity of beta-alkylated monohydric primary alcohol(s) O1) / (total quantity of alkyl ester(s) of beta-alkylated monohydric primary alcohol(s) O2), is preferably comprised between 20/80 and 80/20.

Preferably, the ratio (total quantity of beta-alkylated monohydric primary alcohol(s) O1) / (total quantity of alkyl ester(s) of beta-alkylated monohydric primary alcohol(s) O2), is comprised between 25/75 and 75/25.

An effective amount of the product of the invention sufficient to destabilize a foam, *e.g.* to reduce the formation of a foam or to break an existing foam, is preferably comprised between 100 ppm and 5000 ppm, more preferably between 500 and 5000 ppm.

The invention also concerns a composition comprising:
- a beta-alkylated monohydric primary alcohol O1;
- an alkyl ester of beta-alkylated monohydric primary alcohol O2;
- water;
wherein each beta-alkylated monohydric primary alcohol O1 and O2, identical or different, comprises from 8 to 16 carbon atoms; and
wherein the alkyl of the alkyl ester of beta-alkylated monohydric primary alcohol O2 comprises from 5 to 12 carbon atoms.

The beta-alkylated monohydric primary alcohol(s) O1 and the alkyl ester(s) of beta-alkylated monohydric primary alcohol(s) O2 are as described above, including preferential and advantageous features.

The total quantity of beta-alkylated monohydric primary alcohol(s) O1 and of alkyl ester(s) of beta-alkylated monohydric primary alcohol(s) O2, is of at least 0.01% by weight, more preferably at least 0.05% by weight based on the weight of the composition.

Composition of the invention, wherein the ratio (total quantity of beta-alkylated monohydric primary alcohol(s) O1) / (total quantity of alkyl ester(s) of beta-alkylated monohydric primary alcohol(s) O2), is preferably comprised between 20/80 and 80/20.

Preferably, the ratio (total quantity of beta-alkylated monohydric primary alcohol(s) O1) / (total quantity of alkyl ester(s) of beta-alkylated monohydric primary alcohol(s) O2), is comprised between 25/75 and 75/25.

The composition of the invention may further comprise a compound capable of foaming the composition.

The compound capable of foaming the composition may be any residue of an industrial process that induces foam formation, such as a surfactant used in lubrication or in emulsification.

The quantity of compound capable of foaming the composition is preferably of at least 0.05 % by weight, more preferably of at least 0.1 % by weight, based on the weight of the composition.

Preferably, the quantity of compound capable of foaming the composition is at most 5% by weight, more preferably at most 1% by weight, even more preferably at most 0.5% by weight based on the weight of the composition.

Water may be tap water, distilled water or a brine.

The quantity of water is preferably of at least 50% by weight based on the weight of the composition.

A process for preparing the composition of the invention, comprises the step of bringing together a beta-alkylated monohydric primary alcohol O1, an alkyl ester of beta-alkylated monohydric primary alcohol O2 and water, and optionally a compound capable of foaming the composition; wherein each beta-alkylated monohydric primary alcohol O1 and O2, identical or different, comprises from 8 to 16 carbon atoms, and wherein the alkyl of the alkyl ester of beta-alkylated monohydric primary alcohol O2, comprises from 5 to 12 carbon atoms.

The beta-alkylated monohydric primary alcohol O1 and the alkyl ester of beta-alkylated monohydric primary alcohol O2 are first brought together or independently mixed to water according to their simultaneous or separate use in accordance with embodiments described above for the process of preparation of the product of the invention.

The invention also concerns a method for destabilizing a foam of a composition, by contacting the foam with a beta-alkylated monohydric primary alcohol O1 and an alkyl ester of beta-alkylated monohydric primary alcohol O2; wherein each beta-alkylated monohydric primary alcohol O1 and O2, identical or different, comprises from 8 to 16 carbon atoms, and wherein the alkyl of the alkyl ester of beta-alkylated monohydric primary alcohol O2 comprises from 5 to 12 carbon atoms.

The contact between the foam and the alcohol and ester components may be achieve by adding components at the surface of the foam or by adding the components into the composition generating the foam.

The invention also relates to a method for reducing foam formation of a composition, by adding into the composition a beta-alkylated monohydric primary alcohol O1 and an alkyl ester of beta-alkylated monohydric primary alcohol O2; wherein each beta-alkylated monohydric primary alcohol O1 and O2, identical or different, comprises from 8 to 16 carbon atoms, and wherein the alkyl of the alkyl ester of beta-alkylated monohydric primary alcohol O2 comprises from 5 to 12 carbon atoms.

The invention further relates to a method for breaking a foam of a composition by contacting the foam with a beta-alkylated monohydric primary alcohol O1 and an alkyl ester of beta-alkylated monohydric primary alcohol O2; wherein each beta-alkylated monohydric primary alcohol O1 and O2, identical or different, comprises from 8 to 16 carbon atoms, and wherein the alkyl of the alkyl ester of beta-alkylated monohydric primary alcohol O2 comprises from 5 to 12 carbon atoms.

In methods according to the invention, the beta-alkylated monohydric primary alcohol(s) O1 and the alkyl ester(s) of beta-alkylated monohydric primary alcohol(s) O2 are as described above, including preferential and advantageous features.

Preferably, in methods according to the invention, the composition is a composition comprising water, in particular at a content of at least 50% by weight based on the weight of the composition.

Advantageously, in methods of the invention, the ratio (total quantity of beta-alkylated monohydric primary alcohol(s) O1) / (total quantity of alkyl ester(s) of beta-alkylated monohydric primary alcohol(s) O2), is preferably comprised between 20/80 and 80/20.

Preferably, the ratio (total quantity of beta-alkylated monohydric primary alcohol(s) O1) / (total quantity of alkyl ester(s) of beta-alkylated monohydric primary alcohol(s) O2), is comprised between 25/75 and 75/25.

Advantageously, in methods of the invention, the total quantity of beta-alkylated monohydric primary alcohol(s) O1 and of alkyl ester(s) of beta-alkylated monohydric primary alcohol(s) O2, is of at least 0.01% by weight based on the weight of the composition.

Preferably, the total quantity of beta-alkylated monohydric primary alcohol(s) O1 and of alkyl ester(s) of beta-alkylated monohydric primary alcohol(s) O2, is of at least 0.05% by weight based on the weight of the composition.

Preferably, the total quantity of beta-alkylated monohydric primary alcohol(s) O1 and of alkyl ester(s) of beta-alkylated monohydric primary alcohol(s) O2 is of at most 0.5% by weight based on the weight of the composition.

The invention is further described in the following examples. It will be appreciated that the invention as claimed is not intended to be limited in any way by these examples.

### Example 1: Preparation of beta-alkylated monohvdric primary alcohols and esters thereof

### 1.1. Chemicals used

- Primary alcohols:
   ∘ 3-methylbutanol from Tradebe;
   ∘ Octanol, Ecorol 8/98 from Ecogreen Oleochemicals;
- beta-alkylated monohydric primary alcohols:
   ∘ 2-ethylhexanol (C8) from Perstorp;
   ∘ 2-propylheptanol (C10) from Perstorp.

### 1.2. Preparation of Guerbet alcohols

2-isopropyl-5-methylhexanol (C10iso) and 2-hexyldecanol (C16) were prepared from respectively 3-methylbutanol and octanol as primary alcohols according to a Guerbet reaction. Such a reaction is described in patent US 4,518,810 using KOH as a base and palladium as catalyst. The reaction medium were heated up to their respective boiling point.

Each crude reaction mixture was washed several times with demineralized water to remove all of the soaps. The washed products were subsequently filtered and dried under vacuum. Each remaining starting primary alcohol was separated by distillation. Then, each Guerbet alcohol was isolated by distillation.

The Guerbet alcohols thus obtained had each a purity of 98%+/- 1.

### 1.3. Preparation of alkyl ester of beta-alkylated monohydric primary alcohols

2-ethylhexyl hexanoate and 2-propylheptyl hexanoate were prepared by esterification of hexanoic acid with a molar excess (30%) of respectively 2-ethylhexanol and 2-propylheptanol, in presence of methane sulfonic acid, at 150°C, until the acid value reached 3, the acid value being measured according to standard AOCS Cd 3D-63.

Then the excess of each primary alcohol was distilled.

### Example 2: Synergistic effect of the product according to the invention on foaming characteristics of compositions according to the invention

### 2.1 Preparation of additive packages 1-8 according to the invention

Additive package 1-8 were prepared at room temperature by mixing a beta-alkylated monohydric primary alcohol and an ester of beta-alkylated monohydric primary alcohol manually to attain homogeneity.

Contents of each additive package are described in Table 1 below:

**Table 1: Contents of additive packages 1-8 according to the invention**

| | beta-alkylated monohydric primary alcohol (wt%*) | | | | Ester of beta-alkylated monohydric primary alcohol (wt%*) | |
|---|---|---|---|---|---|---|
| | C8 | C10 | C10iso | C16 | 2-ethylhexyl hexanoate | 2-isopropyl-5-methylhexyl hexanoate |
| Additive package 1 | 80 | | | | 20 | |
| Additive package 2 | 50 | | | | 50 | |
| Additive package 3 | | 50 | | | 50 | |
| Additive package 4 | | 75 | | | 25 | |
| Additive package 5 | | | 25 | | 75 | |
| Additive package 6 | | | 50 | | 50 | |
| Additive package 7 | | | | 25 | 75 | |
| Additive package 8 | 75 | | | | | 25 |

| | | | | | | |
|---|---|---|---|---|---|---|
| *based on the weight of the additive package | | | | | | |

### 2.2 Preparation of compositions according to the invention

### 2.2.1 With the use of the additive package according to the invention

Compositions 1-8 according to the invention were prepared by adding 0.5 wt% of a foaming agent (sodium dodecylbenzene sulfonate) and respectively 0.1 wt% of additive package 1-8 according to the invention, into tap water; weight percentages being based on the weight of each composition.

Composition 5.5 according to the invention was prepared as described for compositions 1-8, using 0.05 wt% of additive package 5 according to the invention, instead of 0.1 wt%.

### 2.2.2 Without the use of the additive package according to the invention

Composition 2bis according to the invention was prepared by adding 0.5 wt% of a foaming agent (sodium dodecylbenzene sulfonate) into tap water, followed by the introduction of 0.05 wt% of 2-ethylhexanol and then 0.05 wt% of 2-ethylhexyl hexanoate; weight percentages being based on the weight of the composition.

### 2.3 Preparation of comparative compositions

Comparative compositions 1-4 and 7-8 were prepared as compositions of the invention, by adding 0.5 wt% of a foaming agent (sodium dodecylbenzene sulfonate), and 0.1 wt% of respectively 2-ethylhexyl hexanoate, 2-ethylhexanol, 2-propylheptanol, 2-isopropyl-5-methylhexanol, 2-hexyldecanol and 2-isopropyl-5-methylhexyl hexanoate, into tap water.

Comparative compositions 5 and 6 were prepared as compositions of the invention, by adding 0.5 wt% of a foaming agent (sodium dodecylbenzene sulfonate), and 0.05 wt% of respectively, 2-isopropyl-5-methylhexanol and 2-ethylhexyl hexanoate into tap water.

All weight percentages are based on the weight of the corresponding comparative composition.

### 2.4 Antifoaming property of product according to the invention

The method used to determine the efficiency of the product of the invention as antifoam agent is based on ASTM D892.

A blank sample was prepared by adding 0.5 wt% of a foaming agent (sodium dodecylbenzene sulfonate) into tap water.

200 mL of each sample (compositions of the invention, comparative compositions and blank), were respectively introduced in a 1000 mL graduated cylinder. Each cylinder was then placed in a bath at 24°C. Air was injected in each sample at a flow rate of 94 mL/min for 5 minutes and then the samples were allowed to settle for 10 minutes.

The volumes of foam were measured just after the air flow stopped and after the 10 minutes period.

Results are summarized in Table 2 below:

**Table 2: Foaming characteristics of compositions of the invention and of comparative compositions**

| | | Volumes of foam (mL) | |
|---|---|---|---|
| | Antifoam | 0 min | 10 min |
| Blank | - | 600 | 485 |
| Comparative composition 1 | 0.1% of 2-ethylhexyl hexanoate | 500 | 105 |
| Comparative composition 2 | 0.1% of 2-ethylhexanol | 470 | 43 |
| Composition 1 | 0.1% of additive package 1 | 305 | 0 |
| Composition 2 | 0.1% of additive package 2 | 200 | 0 |
| Composition 2bis | 0.05% of 2-ethylhexanol and 0.05% of 2-ethylhexyl-hexanoate | 310 | 0 |
| Comparative composition 3 | 0.1% of 2-propylheptanol | 550 | 200 |
| Composition 3 | 0.1% of additive package 3 | 190 | 0 |
| Composition 4 | 0.1% of additive package 4 | 300 | 0 |
| Comparative composition 4 | 0.1% of 2-isopropyl-5-methylhexanol | 350 | 20 |
| Composition 5 | 0.1% of additive package 5 | 230 | 0 |
| Composition 6 | 0.1% of additive package 6 | 290 | 10 |
| Comparative composition 5 | 0.05% of 2-isopropyl-5-methylhexanol | 370 | 20 |
| Comparative composition 6 | 0.05% of 2-ethylhexyl hexanoate | 510 | 210 |
| Composition 5.5 | 0.05% of additive package 5 | 295 | 0 |
| Comparative composition 7 | 0.1% of 2-hexvldecanol | 570 | 430 |
| Composition 7 | 0.1% of additive package 7 | 430 | 90 |
| Comparative composition 8 | 0.1% of 2-isopropyl-5-methylhexyl hexanoate | 490 | 30 |
| Composition 8 | 0.1% of additive package 8 | 465 | 15 |

It can be observed with the blank sample, that without any antifoam, a lot of foam is produced: 600 mL of foam at t=0 and still 485 mL after 10 min of rest.

Use of a beta-alkylated monohydric primary alcohol or an alkyl ester of beta-alkylated monohydric primary alcohol as antifoam reduces the volume of foam formed at t=0, from -5% with 2-hexyldecanol (comparative composition 7) to -41% with 2-isopropyl-5-methylhexanol (comparative composition 4).

But when a combination of a beta-alkylated monohydric primary alcohol and an alkyl ester of beta-alkylated monohydric primary alcohol, wherein each beta-alkylated monohydric primary alcohol, identical or different, comprises from 8 to 16 carbon atoms, and wherein the alkyl of alkyl ester of beta-alkylated monohydric primary alcohol comprises 6 carbon atoms, is added to a composition comprising water and a foaming agent, then the volume of foam is more largely reduced.

For instance, by comparing foam formation at t=0 of blank and of comparative compositions 1 (-16%) and 4 (-41%) with compositions 5 (-61%) or 6 (-51%), we can observed than less foam is formed with compositions comprising the combination of specific beta-alkylated monohydric primary alcohol and specific ester of beta-alkylated monohydric primary alcohol.

The combination of a beta-alkylated monohydric primary alcohol and an alkyl ester of beta-alkylated monohydric primary alcohol, wherein each beta-alkylated monohydric primary alcohol, identical or different, comprises from 8 to 16 carbon atoms, and wherein the alkyl of the alkyl ester of beta-alkylated monohydric primary alcohol comprises 6 carbon atoms, presents a better efficiency than each compound used individually. Consequently, there is a synergistic effect on the reduction of foam formation.

More particularly, the foam formed is not stable and disappears after 4 min by using 0.1 wt% of additive package 2, *e.g.* the combination 2-ethylhexanol/2-ethylhexyl hexanoate at a weight ratio 50/50. By using 0.1 wt% of 2-ethylhexanol or of 2-ethylhexyl hexanoate, there is respectively 43 mL (comparative composition 2) and 105 mL (comparative composition 1) of foam after 10 min.

The foam disappears in 2 min with 0.1 wt% of additive package 5, *e.g.* the combination 2-isopropyl-5-methylhexanol /2-ethylhexyl hexanoate at a weight ratio 25/75. By using 0.1 wt% of 2-isopropyl-5-methylhexanol (comparative composition 4), there is respectively 20 mL of foam after 10 min.

With only 0.05 wt% of additive package 5 (composition 5.5), there is no more foam after 4 min.

The same synergistic effect on the reduction of foam formation can be observed when the two components of the product of the invention are added separately in the aqueous composition. The foam volume of composition 2bis comprising the two components in proportions similar to composition 2 comprising the additive package 2, is reduced by 48% at t=0 and by 100% at t=10min, compared to the blank. More particularly, the foam collapses within 7 minutes.

All those results demonstrate that the combination of a beta-alkylated monohydric primary alcohol, and an alkyl ester of beta-alkylated monohydric primary alcohol, each beta-alkylated monohydric primary alcohol comprising from 8 to 16 carbon atoms and wherein the alkyl of the alkyl ester of beta-alkylated monohydric primary alcohol comprises from 5 to 12 carbon atoms, is a good antifoam.

### 2.5 Defoaming property of composition 2 according to the invention

The method used to determine the efficiency of the product of the invention as defoamer is based on ASTM D892.

Two samples of 200 mL of tap water comprising 0.5 wt% of a foaming agent (sodium dodecylbenzene sulfonate) were each placed into a 1000 mL graduated cylinder which were then placed in a bath at 24°C.

Air was injected into the sample at a flow rate of 94 mL/min for 5 minutes. Once the injection of air stopped, 0.1 wt% of additive package 5 according to the invention was added on the foam formed of one sample, to form composition 5 of the invention.

All weight percentage are based on the weight of the corresponding composition.

The sample which do not received a product of the invention corresponds to the blank.

The volume of foam of blank sample was measured just after the air flow stopped (t=0) and for 7 minutes.

The volume of the foam of composition 5 was measured just after addition of the additive package 5 (t=0) and monitored for 7 minutes.

Data are gathered in Table 3 below.

**Table 3: Defoaming properties of composition 5 of the invention**

| | | Volumes of foam (mL) | | | |
|---|---|---|---|---|---|
| | Defoamer | 0 min | 3 min | 5 min | 7min |
| Blank | - | 600 | 550 | 540 | 525 |
| Composition 5 | 0.1% of additive package 5 | 575 | 240 | 40 | 0 |

It can be seen that composition 5 of the invention comprising 0.1% of a mixture of 25 wt% of 2-isopropyl-5-methylhexanol and 75 wt% of 2-ethylhexyl hexanoate, can break an existing foam within 7 minutes.

The combination of a beta-alkylated monohydric primary alcohol, and an alkyl ester of beta-alkylated monohydric primary alcohol, each beta-alkylated monohydric primary alcohol comprising independently from 8 to 16 carbon atoms and wherein the alkyl of the alkyl ester of beta-alkylated monohydric primary alcohol comprises from 5 to 12 carbon atoms, may be used as a defoamer.

### Example 3: Importance of each component in the product of the invention

### 3.1 Preparation of comparative additive packages 1-4

To evaluate the importance of the specific alcohol and the specific ester in the product of the invention, comparative additive packages 1-4 were prepared. Their contents are described in Table 4 below:

**Table 4: Contents of comparative additive packages 1-4**

| | Alcohol (wt%) | | Ester (wt%) | | | |
|---|---|---|---|---|---|---|
| Comparative additive package | 2-octanol | 2-ethylhexanol | 2-ethylhexyl hexanoate | sorbitan monooleate | glycerol monooleate | glycerol mono caprylate |
| 1 | 50 | | 50 | | | |
| 2 | | 50 | | 50 | | |
| 3 | | 50 | | | 50 | |
| 4 | | 50 | | | | 50 |

| | | | | | | |
|---|---|---|---|---|---|---|
| *based on weight of the comparative additive package | | | | | | |

### 3.2 Importance of the beta-alkylated monohydric primary alcohol

To evaluate the importance of the beta-alkylated monohydric primary alcohol comprising from 8 to 16 carbon atoms, two comparative compositions 9-10 were prepared as described in Example 2.2.1 by using as antifoam, instead of an additive package of the invention, respectively 0.1 wt% of 2-octanol and 0.1 wt% of a comparative additive package 1 comprising 50 wt% of 2-octanol and 50 wt% of 2-ethylhexyl hexanoate.

Foam formation of both compositions were tested as in Example 2.4.

Results are summarized in Table 5 below.

It can be seen that there was no synergistic effect between 2-octanol and 2-ethylhexyl hexanoate. 2-octanol reduces more foam when used alone.

### 3.3 Importance of the alkyl ester of beta-alkylated monohydric primary alcohol

To evaluate the importance of the alkyl ester of beta-alkylated monohydric primary alcohol, wherein the beta-alkylated monohydric primary alcohol comprises from 8 to 16 carbon atoms and the alkyl of the alkyl ester of beta-alkylated monohydric primary alcohol comprises from 5 to 12 carbon atoms, three comparative compositions 12, 14 and 16, were prepared as described in Example 2.2.1 by using as antifoam, instead of an additive package of the invention, respectively 0.1 wt% of comparative additive packages 2-4.

In parallel, comparative compositions 11, 13 and 15 comprising respectively 0.1 wt% of each ester were prepared.

Results are summarized in Table 5 below.

**Table 5: Foaming properties of comparative compositions**

| | | Volumes of foam (mL) | |
|---|---|---|---|
| | Antifoam | 0 min | 10 min |
| Blank | - | 600 | 485 |
| Comparative composition 1 | 0.1% of 2-ethylhexyl hexanoate | 500 | 105 |
| Comparative composition 9 | 0.1% of 2-octanol | 500 | 70 |
| Comparative composition 10 | 0.1% of comparative additive package 1 | 475 | 105 |
| Comparative composition 2 | 0.1% of 2-ethylhexanol | 470 | 43 |
| Comparative composition 11 | 0.1% sorbitan monooleate | 710 | 595 |
| Comparative composition 12 | 0.1% of comparative additive package 2 | 630 | 245 |
| Comparative composition 13 | 0.1% glycerol monooleate | 565 | 490 |
| Comparative composition 14 | 0.1% of comparative additive package 3 | 580 | 180 |
| Comparative composition 15 | 0.1% glycerol monocaprylate | 595 | 470 |
| Comparative composition 16 | 0.1% of comparative additive package 4 | 500 | 70 |

No synergistic effect was observed with any comparative compositions comprising comparative additive packages 2-4. Each time the comparative composition comprising 0.1 wt% of 2-ethylhexanol, better reduced the foam at t=0 and t=10.

## Claims

1. Product comprising or consisting of:
- a beta-alkylated monohydric primary alcohol O1; and
- an alkyl ester of a beta-alkylated monohydric primary alcohol O2;
wherein each beta-alkylated monohydric primary alcohol O1 and O2, identical or
different, comprises from 8 to 16 carbon atoms; and
wherein the alkyl of the alkyl ester of a beta-alkylated monohydric primary alcohol O2 comprises from 5 to 12 carbon atoms;
as a combined preparation for simultaneous or separate use.

2. Product according to claim 1, wherein each beta-alkylated monohydric primary alcohols O1 and O2 are individually of Formula (I): 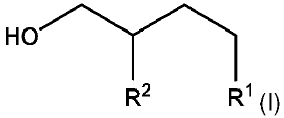 wherein R¹ and R², identical or different, are linear or branched alkyl group comprising from 2 to 6 carbon atoms.

3. Product according to claim 2, wherein R¹ and R² are identical in a beta-alkylated monohydric primary alcohol.

4. Product according to any of claims 1 to 3, wherein the alkyl of the alkyl ester of beta-alkylated monohydric primary alcohol O2 comprises from 6 to 10 carbon atoms.

5. Product according to any of claims 1 to 4, wherein the total quantity of beta-alkylated monohydric primary alcohol(s) O1 is of at least 20% by weight, and the total quantity of alkyl ester(s) of beta-alkylated monohydric primary alcohol(s) O2 is of at least 20% by weight, weight percentages being based on the weight of the product.

6. Product according to any of claims 1 to 5, wherein the ratio (total quantity of beta-alkylated monohydric primary alcohol(s) O1) / (total quantity of alkyl ester(s) of beta-alkylated monohydric primary alcohol(s) O2), is preferably comprised between 20/80 and 80/20.

7. Process for preparing a product according to any of claims 1 to 6, by bringing together a beta-alkylated monohydric primary alcohol O1 and an alkyl ester of beta-alkylated monohydric primary alcohol O2, wherein each beta-alkylated monohydric primary alcohol O1 and O2, identical or different, comprises from 8 to 16 carbon atoms, and wherein the alkyl of the alkyl ester of beta-alkylated monohydric primary alcohol O2, comprises from 5 to 12 carbon atoms.

8. Use of the product according to any of claims 1 to 6, as an antifoam and/or a defoamer.

9. Use according to claim 8, wherein the ratio (total quantity of beta-alkylated monohydric primary alcohol(s) O1) / (total quantity of alkyl ester(s) of beta-alkylated monohydric primary alcohol(s) O2), is preferably comprised between 20/80 and 80/20.

10. A composition comprising:
- a beta-alkylated monohydric primary alcohol O1;
- an alkyl ester of beta-alkylated monohydric primary alcohol O2;
- water;
wherein each beta-alkylated monohydric primary alcohol O1 and O2, identical or different, comprises from 8 to 16 carbon atoms; and
wherein the alkyl of the alkyl ester of beta-alkylated monohydric primary alcohol O2 comprises from 5 to 12 carbon atoms.

11. Method for destabilizing a foam of a composition, by contacting the foam with a beta-alkylated monohydric primary alcohol O1 and an alkyl ester of beta-alkylated monohydric primary alcohol O2; wherein each beta-alkylated monohydric primary alcohol O1 and O2, identical or different, comprises from 8 to 16 carbon atoms, and wherein the alkyl of the alkyl ester of beta-alkylated monohydric primary alcohol O2 comprises from 5 to 12 carbon atoms.

12. Method for reducing foam formation of a composition, by adding into the composition a beta-alkylated monohydric primary alcohol O1 and an alkyl ester of beta-alkylated monohydric primary alcohol O2; wherein each beta-alkylated monohydric primary alcohol O1 and O2, identical or different, comprises from 8 to 16 carbon atoms, and wherein the alkyl of the alkyl ester of beta-alkylated monohydric primary alcohol O2 comprises from 5 to 12 carbon atoms.

13. Method for breaking a foam of a composition by contacting the foam with a beta-alkylated monohydric primary alcohol O1 and an alkyl ester of beta-alkylated monohydric primary alcohol O2; wherein each beta-alkylated monohydric primary alcohol O1 and O2, identical or different, comprises from 8 to 16 carbon atoms, and wherein the alkyl of the alkyl ester of beta-alkylated monohydric primary alcohol O2 comprises from 5 to 12 carbon atoms.

14. Method according to any of claims 11 to 13, wherein the ratio (total quantity of beta-alkylated monohydric primary alcohol(s) O1) / (total quantity of alkyl ester(s) of beta-alkylated monohydric primary alcohol(s) O2), is preferably comprised between 20/80 and 80/20.

15. Method according to any of claims 11 to 14, wherein the total quantity of beta-alkylated monohydric primary alcohol(s) O1 and of alkyl ester(s) of beta-alkylated monohydric primary alcohol(s) O2, is of at least 0.01% by weight based on the weight of the composition.
